# EUROPEAN PATENT APPLICATION

(11) **EP 1 398 131 A1**
(43) Date of publication of application: **17.03.2004**
(21) Application number: 03020618.9
(22) Date of filing: 10.09.2003
(51) Int. Cl.: B29C 41/14, A61M 25/10, A61B 19/04, A61F 6/04

(54) **Dip-molded polymeric medical devices with reverse thickness gradient and dip molding process**

(30) Priority: 12.09.2002 US 241942
(71) Applicant: Polyzen, Inc., Cary, NC 27512 (US)
(72) Inventor: Shah, Tilak M., Cary NC 27511 (US)
(74) Representative: Schüssler, Andrea, Dr.

(57) **Abstract**

An expandable polymeric medical article is formed by a novel dip-molding process, involving multiple dipping and rotational drying of liquid polymeric material layer at different pivotal positions. Preferably, the rotational drying is conducted so as to form an expandable polymeric medical article having a reverse thickness gradient in relation to the thickness gradient of conventional dip-molded polymeric medical articles. Specifically, the expandable polymeric medical article of the present invention has an expandable body member and a neck member, wherein such neck member has a wall thickness that is greater than that of the expandable body member, before any significant expansion of such body member.

## Description

### BACKGROUND OF THE INVENTION

### Field Of The Invention

The present invention generally relates to expandable polymeric medical articles and method for forming the same. Such expandable polymeric medical articles broadly include, but are not limited to, endometrial ablation balloons, low-pressure catheter balloons, medication directing/delivery tubing, fluid storage/dispensing compartments, protective covers, etc.

### Description of the Related Art

The Food and Drug Administration (FDA) has recently approved endometrial ablation procedures for treating menorrhagia. Such procedures use freezing and/or heat to destroy a layer of tissue on the inner wall of uterus, so as to stop the excessive menstrual flow. In comparison with the conventional hysterectomy approach that removes the whole uterus, endometrial ablations are less invasive, and more and more menorrhagia patients will be treated with endometrial ablations in the near future.

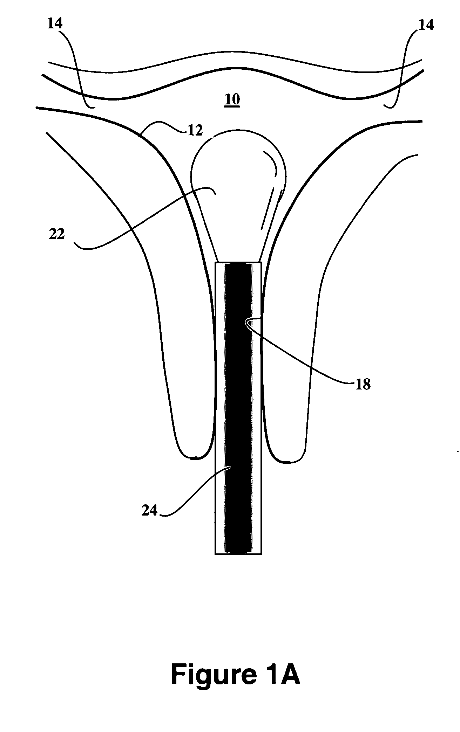

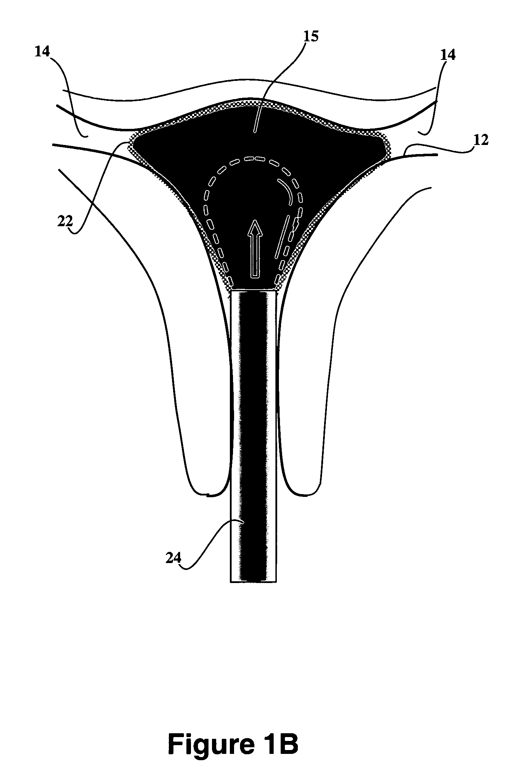

The ThermaChoice® Uterine Balloon Therapy provided by Johnson & Johnson's Gynecare Division involves inserting an expandable balloon 22 into the uterus cavity 10 through the cervical canal 18, inflating/heating said balloon with heated fluid 15, and pressing the heated balloon against the uterine wall 12, so as to destroy an inner layer of tissue on such uterine wall 12, as shown in Figures 1A and 1B.

The expanded balloon 22 has to fill the uterine cavity and conform to the expanded inner surface of such uterine cavity, as shown in Figure 1B. Portions of the balloon 22, namely the body portion near the distal end, undergo very extensive expansion and stretch all the way into the entrance to the fallopian tubes 14. Therefore, the balloon 22 at such body portion near the distal end with respect to the inserting catheter 24) has to be sufficiently thin, in order to allow such extensive expansion at a predetermined inflation pressure. If the balloon 22 at such body portion near the distal end is too thick, a much higher inflation pressure is required for achieving the desired degree of expansion, resulting in increased operating cost and higher risk associated with use of the higher inflation pressure. Currently available balloons do not expand sufficient at their body portions, in spite of high pressure applied thereto, and they therefore are incapable of covering the entire uterine cavity.

On the other hand, the neck portion of the balloon 22 near the proximal end, which functions to affix the balloon 22 to the inserting catheter 24 for insertion into and withdrawal from the uterine cavity 10, has to be sufficiently thick, in order to provide the required structural integrity between the balloon 22 and the inserting catheter 24. If the balloon 22 at such neck portion is too thin, the balloon 22 is vulnerable of breaking off the inserting catheter 24, when the inserting catheter 24 withdraws the inflated balloon 22 from the uterine cavity 10.

It is therefore an object of the present application to form a catheter balloon, especially an endometrial ablation balloon or like articles, which has a relatively thick neck portion at a proximal end and a relatively thin body portion at a distal end, so as to allow sufficient expansion of the body portion, as well as to provide structural integrity for the balloon-catheter attachment.

Conventional catheter balloons are formed either by blow molding processes, or by dip molding processes.

Blow molding involves formation of a straight elastomic tube, and axial/radial expansion of such straight elastomic tube at a medial working section to form a balloon. The balloon so formed is thinner in the medial working section due to the axial/radial expansion, and is thicker at its two ends where much less expansion has taken place. For more details about the blow molded balloons, see U.S. Patent No. 5,826,588 and U.S. Patent Application Publication No. 2002/0072707.

However, the balloon formed by such blow molding process has already undergone substantial expansion at the medial working section, and it suffers from high strain and stress resulted from such expansion. Such blow-molded balloon therefore is not suitable for further extensive expansion, which is generally required for balloons used in endometrial ablation procedures. As a result, the blow-molded balloon is not ideal for endometrial ablation usage as described hereinabove.

Dip molding, on the other hand, avoids any significant expansion of the balloon body during the manufacturing process. The balloon formed by such dip molding process therefore does not suffer from strain and stress associated with such expansion, and the dip-molded balloon can be used as endometrial ablation balloons to undergo the necessary extensive expansion in the uterine cavity. For example, U.S. Patent No. 5,562,720 discloses use of dip-molded balloons for endometrial ablation procedure.

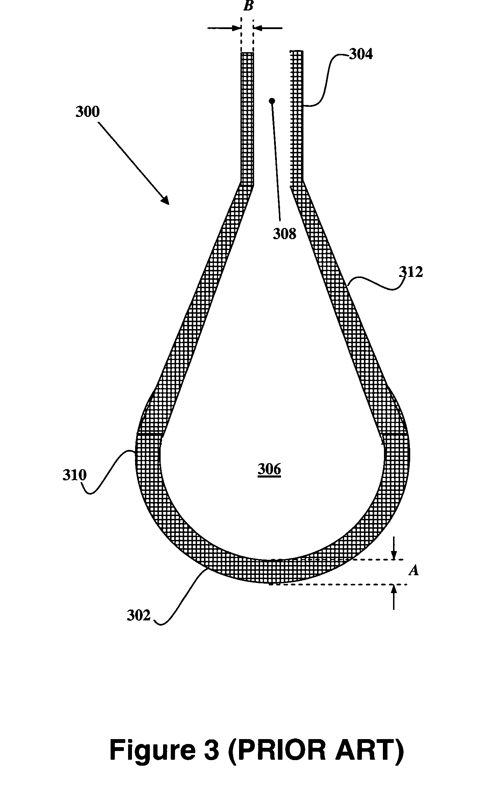

However, the balloons formed by dip molding process are generally thicker at their body portion and thinner at their neck portion. For example, Figure 3 shows a conventional dip-molded balloon 300, having a body portion 302 and a neck portion 304.

The balloon 300 is formed by dipping a mandrel of desired shape into a polymeric solution, so as to obtain a layer of liquid polymeric material coated over the outer surface of the mandrel, drying and curing such layer of polymeric material on such mandrel, and then peel such layer of polymeric material off the mandrel to form a seamless dip-molded balloon of desired shape. During the drying and curing steps, the liquid polymeric coating tends to flow from the neck of the mandrel down to the bottom of the mandrel body under the effect of gravity force, resulting in a dip-molded balloon that is thicker at its body portion near the bottom, but thinner at its neck portion, as shown in Figure 3.

Specifically, the conventional dip-molded balloon 300 comprises an expandable enclosure 306 including a distal body portion 302, and a proximal neck portion 304, wherein the distal body portion 302 has a first wall thickness **A**, wherein the proximal neck portion defines an opening 308 communicatively connected to the expandable enclosure 306 and has a second wall thickness **B**, and wherein **A** is greater than **B**. Such balloon 300 is characterized by a thickness gradient, which gradually increases from the thin proximal neck portion 304, through the thicker transition portion 312 and the thicker equatorial portion 310, and to the thickest distal body portion 302. Such thickness gradient results from slow flowing of the liquid polymeric material along the direction of gravity during the drying and curing processes, i.e., from the proximal (i.e., upper) neck portion 304 through the transition portion 312 and the equatorial portion 310 to the distal (i.e., lower) body portion 302.

The conventional dip-molded balloon, having a thick body portion and a thin neck portion, is not suitable for performing endometrial ablation, because the thick body portion of such balloon, when expanded under the pressure of air or a hot liquid, requires a significantly high expansion pressure to achieve the desired degree of expansion, while the thin neck portion of such balloon, under such high expansion pressure, is likely to rupture or breakout, resulting in catastrophic disconnection of such balloon from the inserting catheter.

It is therefore an object of the present invention to provide a dip-molded balloon that does not have the above-described disadvantages of conventional dip-molded balloons, and that is suitable to be used as endometrial ablation balloon.

Several references teach elimination or reduction of the thickness gradient described hereinabove that is characteristic to dip-molded balloons or like polymeric medical articles (such as condoms or surgical gloves), so as to achieve even thickness in different portions of the balloons or like articles. For example, U.S. Patent No. 6,329,444 discloses production of a surgical glove using dip-molding process, which is rotated during the drying process to achieve an even distribution of the dipping solution, so as to eliminate such thickness gradient. U.S. Patent No. 5,091,442 discloses manufacturing of a condom using dip-molding process, wherein a glass former is rotated and agitated during the drying process to obtain an evenly distributed latex coating over such former, for purpose of eliminating the thickness gradient that is commonly seen in the dipped-molded articles.

However, no prior art references has appreciated the need for, much less has taught or suggested formation of, a dip-molded balloon or like polymeric medical article, having a thickness gradient that is reversed with respect to the above-described thickness gradient characterizing conventional dip-molded balloon.

Reverse thickness gradient is defined herein as a relatively thick neck portion at a proximal end of a dip-molded balloon or like polymeric medical article, and a relatively thin body portion at a distal end of such dip-molded balloon or like polymeric medical article.

The present invention is based on the discovery that a dip-molded balloon with such reverse thickness gradient is especially useful for endometrial ablation procedure, wherein the thinner body portion of such balloon, formed free of expansion and stress associated therewith, allows extensive expansion for covering and pressing the uterine wall under relatively low expansion pressure, and wherein the thicker neck portion of such balloon provides structural integrity for the attachment of such balloon to the inserting catheter.

It is therefore an object of the present invention to form a dip-molded balloon or like polymeric medical article with such reverse thickness gradient.

### SUMMARY OF THE INVENTION

One aspect of the present invention relates to a dip-molded polymeric medical article, comprising an expandable enclosure including a distal portion, and a neck including a proximal portion, wherein such distal portion has a first wall thickness before expansion, wherein said proximal portion includes an opening communicatively connected to such expandable enclosure and has a second wall thickness, and wherein the second wall thickness is greater than the first wall thickness.

Another aspect of the present invention relates to a dip-molded polymeric medical article, comprising an expandable body member and at least one neck member connected thereto, wherein the at least one neck member is characterized by:
(1) a cross-sectional diameter that is smaller than that of the expandable body member before expansion, and
(2) a wall thickness that is greater than that of the expandable body member before expansion.

Yet another aspect of the present invention relates to an expandable polymeric medical article, formed by:
dipping a mandrel into a solution of a polymeric material for one or more times to apply a layer of polymeric material onto the mandrel, and
subsequently solidifying such layer of the polymeric material on the mandrel to form an expandable polymeric medical article,
wherein the mandrel comprises a first end and a second end, while the first end contacts the polymeric solution before the second end does during the dipping process, wherein the expandable polymeric medical article so formed comprises a first portion that is solidified on the first end of the mandrel, and a second portion that is solidified on the second end of the mandrel, the second portion having a wall thickness greater than that of the first portion before expansion of such expandable polymeric medical article.

A still further aspect of the present invention relates to a dip-molding method for forming an expandable polymeric medical article, comprising the following steps:
(a) providing a liquid dip-molding composition comprising at least one polymeric material;
(b) providing a rotatory mandrel capable of axial rotation at an adjustable rotational speed, wherein such rotatory mandrel is capable of being pivotally rotated in at least one additional direction;
(c) dipping the rotatory mandrel into the liquid dip-molding composition so as to apply a layer of the polymeric material onto such rotatory mandrel;
(d) removing the rotatory mandrel from the liquid dip-molding composition;
(e) repeating steps (c) to (d) for one or more times, wherein between each dipping, the layer of polymeric material is partially dried on the rotatory mandrel at a substantially vertical position, with the rotatory mandrel axially rotating during such drying;
(f) pivotally rotating the rotatory mandrel in such additional direction to a substantially inverted position, and partially drying the layer of polymeric material thereat with such rotatory mandrel axially rotating during the drying;
(g) optionally, pivotally rotating the rotatory mandrel in such additional direction to a substantially horizontal position, and partially drying the layer of polymeric material thereat with such rotatory mandrel axially rotating during the drying;
(h) optionally, repeating steps (c) to (g) until said layer of polymeric material achieves a predetermined average thickness;
(i) curing said layer of polymeric material on said rotatory mandrel; and
(j) removing said layer of polymeric material from the rotatory mandrel to form said expandable polymeric medical article.

Various other aspects, features and embodiments of the invention will be more fully apparent from the ensuing disclosure and claims.

### BRIEF DESCRIPTION OF THE DRAWINGS

Figures 1A and 1B is perspective view of an endometrial ablation balloon inserted into a uterus cavity, before and after expansion, respectively.

Figure 2 shows a cross-sectional view of a dip-molded balloon, according to one embodiment of the present invention.

Figure 3 shows a cross-sectional view of a conventional dip-molded balloon.

### DETAILED DESCRIPTION OF THE INVENTION, AND PREFERRED EMBODIMENTS THEREOF

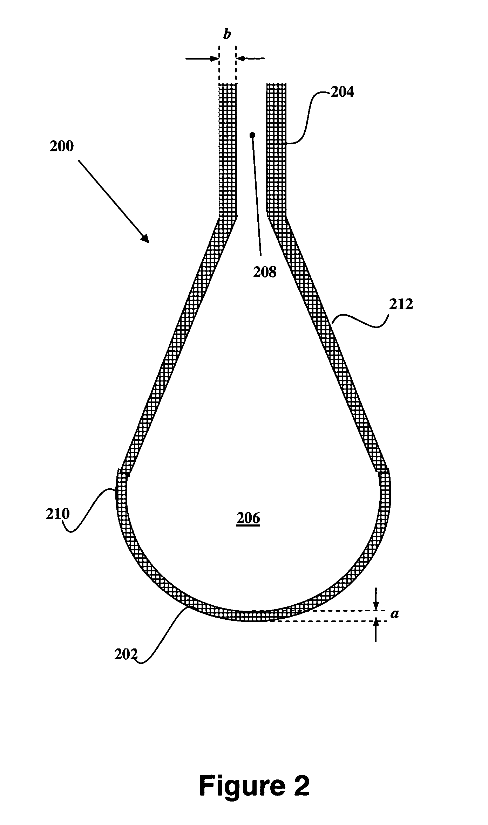

The present invention provides a dip-molded balloon 200, as shown in Figure 2, comprising an expandable enclosure 206 including a distal body portion 202, and a proximal neck portion 204, wherein the distal body portion 202 has a first wall thickness **a**, wherein the proximal neck portion defines an opening 208 communicatively connected to the expandable enclosure 206 and has a second wall thickness **b**, and wherein **b** is greater than **a**. Such balloon 200 is therefore characterized by a reverse thickness gradient, which gradually decreases from the thick proximal neck portion 204, through the thinner transition portion 212 and the thinner equatorial portion 210, and to the thinnest distal body portion 202.

In a specific embodiment of the present invention, the balloon 200 is a silicone endometrial ablation balloon having a neck thickness within the range of from about 6 to 10 mils, a transition wall thickness of about 5 to 7 mils, an equatorial wall thickness of about 3 to 5 mils, and a dome (i.e., the body portion need the bottom) thickness of about 2.5 to 5.5 mils, preferably about 2.5 to 4.5 mils.

Such dip-molded balloon 200 can be formed by any polymeric material having suitable expansion characteristics, such as silicone, polyurethane, cis-1,4 polyisoprene, polyvinyl, SIS elastomer, SIBS elastomer, latex rubber, nitril rubber, butal rubber, etc. Preferably, such dip-molded balloon is formed by a hypo-allergenic material, such as silicone or polyurethane. More preferably, such dip-molded balloon is formed by a silicone material, and most preferably polydimethylsiloxane.

Such dip-molded balloon 200 can have a body portion of any suitable shape or conformation, not limited to the teardrop shape depicted by Figure 2 as an illustrative example. For instance, the body portion of the dip-molded balloon can have a geometrically regular shape, such as spherical, oval, cubic, rectangular, polyhedral, etc., or any geometrically irregular shape. Preferably, the body portion of the dip-molded balloon 200 has a shape that substantially conforms to the specific body cavity into which such balloon is inserted, or the specific body part over which such balloon is covering.

The surface morphology of such balloon is preferably continuous and smooth, free of air bubbles and pores.

The dip-molded balloon of the present invention as described hereinabove can be extrapolated to any dip-molded polymeric medical article, which comprises an expandable body member of a larger cross-section diameter and at least one neck member of a smaller cross-section diameter that is connected to such expandable body member, wherein the neck member has a wall thickness that is greater than that of the expandable body member before any expansion of such polymeric article. Such dip-molded polymeric medical article includes, but is not limited to, condoms, gloves, tamponade device for control of postpartum hemorrhage, medication directing/delivery tubing, fluid storage/dispensing compartments, protective covers, etc.

The dip-molded polymeric medical article of the present invention is formed by a novel dip-molding process that involves multiple dipping and rotational drying of liquid polymeric material layer at different pivotal positions of the mandrel.

Such dip-molding process differs from conventional dip-molding processes, by rotating the mandrel to different pivotal positions in concurrency with the continuous axial rotation of such mandrel during drying of the liquid polymeric material layer coated over the mandrel, so as to achieve a dip-molded product with a desired thickness gradient, which is substantially reverse to that of dip-molded products formed by conventional dip-molding process.

Conventional dip-molding process involves straightly dipping a mandrel into a solution of a polymeric material to apply a layer of the polymeric material onto said mandrel, drying and curing the layer of polymeric material on the mandrel to form a polymeric end product. The mandrel usually has an upper end and a bottom end, while the bottom end contacts the polymeric solution before the upper end does during the dipping. The layer of polymeric material is dried and cured on the mandrel with little or no pivotal rotation of the mandrel, so the polymeric material slowly flows from the upper end to the bottom end under the influence of gravity, resulting in a polymeric end product with a thinner upper portion (as dried on the upper end of the mandrel) and a thicker bottom portion (as dried on the bottom end of the mandrel).

The dip-molding process according to the present invention involves pivotal rotation of the mandrel during the drying and/or curing steps, so to change the flow direction of the polymeric material to achieve polymeric end product with a desired thickness gradient, preferably a reverse thickness gradient, i.e., with a thicker upper portion and a thinner bottom portion.

Specifically, the dip-molding process of the present invention involves the following steps:

### (1) Providing a Liquid Dip-Molding Composition:

Such liquid dip-molding composition comprises at least one polymeric material, such as silicone, polyurethane, polyethylene, polypropylene, polyvinyl, latex, etc., either in its liquid state, if such polymeric material is a liquid, or dissolved or dispersed in a solvent so as to form a liquid dipping solution, if such polymeric material is a solid.

Such liquid dip-molding composition is preferably pseudoplastic, i.e., it is shear-thinable but does not exhibit thixotropy, and it instantaneously decreases in viscosity with increase in shear strain rate.

More preferably, such liquid dip-molding composition comprises silicone. Most preferably, such as liquid dip-molding composition comprises xylene dispersion of dimethyl siloxane. For example, the Implant Grade Dimethyl Silicone Elastomer Dispersion SILBIONE® V40000 commercialized by RHODIA Silicones (Cranbury, NJ), can be used for practicing the present invention. Similar silicone dispersions are also commercialized by Nusil Technology (Carpinteria, CA).

The viscosity of liquid dip-molding composition is generally within a range of from about 400 centipoises to about 1000 centipoises, preferably within a range of from about 500 centipoises to about 900 centipoises, and most preferably within a range of from about 700 centipoises to about 800 centipoises, adjusted either by adding solution or by adding the solid polymeric material.

### (2) Providing Rotatory Mandrel:

The rotatory mandrel employed in the present invention is capable of both axial rotation at an adjustable rotational speed along its axis, and pivotal rotation along at least one additional direction.

Specifically, such rotatory mandrel can perform axial rotation at a first position that is substantially vertical, and it can also be pivotally rotated to, while concurrently axially rotating, a second position that is substantially inverted to its first position, i.e., an upside-down position in relation to the first substantially vertical position.

Such rotation of the mandrel at the second, inverted position will assist redistribution of the liquid polymeric material over the surface of such mandrel. Specifically, the flow of such liquid polymeric material from the neck to the bottom during drying at the first, vertical position is reversed, and such liquid polymeric material starts to flow from the bottom to the neck, due to the inversion of the mandrel. Therefore, rotation of the mandrel at the second, inverted position for a sufficiently long period of time during the drying step will result in a polymeric end product having a thicker neck portion and a thinner bottom portion, as desired and described hereinabove.

Preferably, such rotatory mandrel can further be pivotally rotated to, while concurrently axially rotating, a third position that is substantially horizontal, i.e., a position that is perpendicular to said first, substantially vertical position. Such horizontal rotation will assure the even distribution of the liquid polymeric material along the radial direction of the polymeric end product.

Such rotatory mandrel as described hereinabove can be incorporated into a 3-axises dipping system, which comprises one or more mandrels as well as other necessary mechanical parts for rotating such mandrels both axially and pivotally to the above-mentioned positions. For example, NAVIGATOR® Dipping System provided by ACC Automation Corporation U.S.A. (Akron, OH) provides two axis or three axis rotation.

In a preferred embodiment of the present invention, a dipping system comprising multiple rotatory mandrels as described hereinabove is employed for scale-up commercial production of dip-molded polymeric medical articles of the present invention. For example, such dipping system may comprise at least four rotatory mandrels, more preferably 30 rotatory mandrels, and most preferably above 100 rotatory mandrels.

### (3) Dipping:

The rotatory mandrel is subsequently dipped into the liquid dip-molding composition to obtain a layer of polymeric material that overcoats the mandrel.

In order to obtain an evenly and sufficiently thin coating of the polymer material, the rotatory mandrel is axially rotated at a predetermined rotating speed when dipped into the liquid dip-molding composition. Such axial rotating speed depends on the viscosity of the liquid dip-molding composition. However, if the axial rotating speed is too fast, it will cause surface turbulence in the dip-molding composition and result in formation of air bubbles in the polymeric coating. If the axial rotating speed is too slow, on the other hand, the polymeric coating so formed will be too thick, due to the higher viscosity of the liquid dip-molding composition at a lower shear strain rate. For a dipping composition comprising essentially of xylene dispersion of dimethyl siloxane, the axial rotating speed for dipping is preferably within the range of from about 1 rpm to about 20 rpm, more preferably within the range of from about 5 rpm to about 10 rpm, and most preferably about 8 rpm.

After dipping, the rotatory mandrel is removed from the liquid dip-molding composition, and a layer of polymeric material coating such rotatory mandrel is therefore obtained.

The polymeric coating is preferably formed by multiple dipping, so as to achieve a desired thickness. Between each dipping, the polymeric coating is partially dried to a certain degree, to form a surface layer of sufficient viscosity, for adhesion of next layer of polymeric material during subsequent dipping. The partial drying between each dipping is conducted with the rotatory mandrel 402 axially rotating at a substantially vertical position, as shown in Figure 4A. The axial rotating speed of the rotatory mandrel 402 during such partial drying is within similar range as that used for dipping.

It is important that the drying of the polymeric coating is well controlled, because if it is too dry, air bubbles will form between layers applied during different dipping steps, but if it is not sufficiently dry, the surface layer of the polymeric coating does not provide the necessary adhesion force for obtaining a next polymeric layer of sufficient thickness during subsequent dipping.

Therefore, the drying time and drying temperature between each dipping is desirably optimized for specific polymeric material used in the present invention. For a dipping composition comprising essentially of xylene dispersion of dimethyl siloxane, the drying time between each dipping is preferably within a range of from about 150 seconds to about 200 seconds, when the drying temperature is within a range of from about 70°F to about 90°F. Note that the higher the drying temperature, the shorter the drying time, and vise versa. More preferably, the drying time is within a range of about 180±5 seconds, while the drying temperature is within a range of from about 75°F to about 85°F.

It is preferred that the partial drying of the polymeric coating between multiple dipping steps is conducted sufficiently close to the surface of the liquid dip-molding composition, so that subsequent dipping can be effectuated immediately after the desired degree of dryness is achieve. For example, the partial drying can be conducted at a position about 2 to 4 centimeters above the surface of the liquid dip-molding composition.

### (4) Rotatory Drying at Different Pivotal Positions:

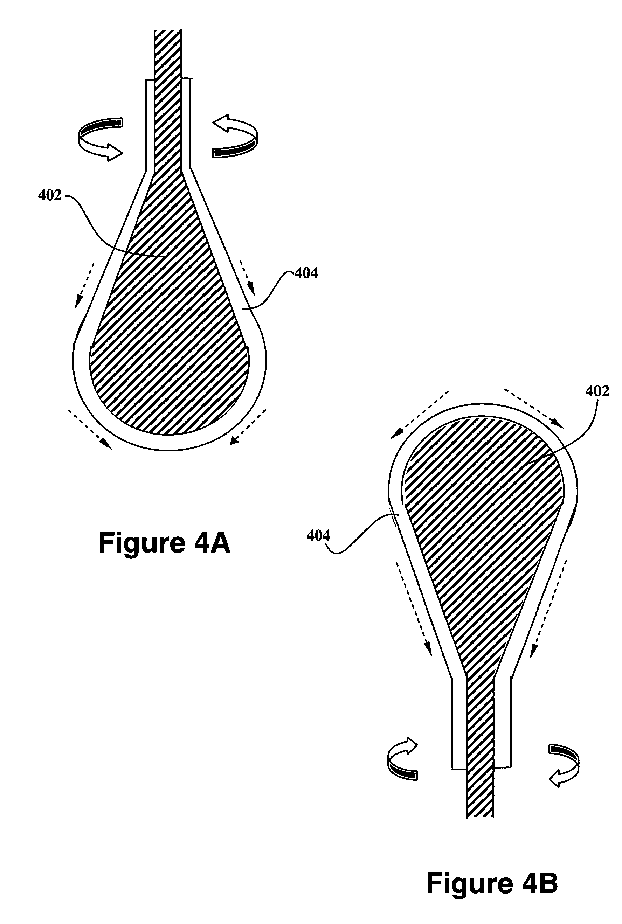

After a polymeric coating of sufficient thickness is obtained through multiple dipping as described hereinabove, the rotatory mandrel 402 is pivotally rotated to a second, inverted position, as shown in Figure 4B. Such second, inverted position is upside-down in relation to the original vertical position shown in Figure 4A, at which the partial drying between dipping is conducted.

During the partial drying between dipping at the vertical position shown in Figure 4A, the polymeric coating 404, which is still in its liquid state, flows slowly from the upper end of the mandrel 402 to the lower end of the mandrel 402 due to gravity, indicated by the arrow heads with dotted lines in Figure 4A.

If the polymeric coating 404 is fully dried in such vertical position, as commonly seen in conventional dip molding processes, the polymeric end product will have a thinner neck, as dried on the upper end of the mandrel 402, and a thicker bottom, as dried on the lower end of the mandrel 402.

However, the dip-molding process of the prevent invention, by pivotally rotating the rotatory mandrel 402 to a second, inverted position before the polymeric coating 404 is fully dried, reverses the flow of the liquid polymeric material during drying, and changes the thickness gradient of the polymeric end product.

Specifically, when the polymeric coating 404 is partially dried at such second, inverted position for a sufficiently long time, sufficient amount of the liquid polymeric material flows from the bottom back to the neck, to form a polymeric end product with a thicker neck and a thinner bottom, as shown in Figure 4B. The drying time of the polymeric coating at such second, inverted position depends on the specific polymeric material used and the drying temperature. For a dipping composition comprising essentially of xylene dispersion of dimethyl siloxane, the drying time is preferably within a range of from about 10 seconds to about 20 seconds, when the drying temperature is within a range of from about 70°F to about 90°F. More preferably, the drying time is preferably within a range of about 15±5 seconds, when the drying temperature is within a range of from about 75°F to about 85°F.

Note that the rotatory mandrel continues to rotate axially at such second, inverted position, so as to ensure even flow of the liquid polymeric material along radial direction of such mandrel. Preferably, such rotatory mandrel is rotating at such second, inverted position with an axial rotation speed that is significantly higher than that used for the partial drying between dipping. For example, such rotatory mandrel rotates at such second, inverted position with an axial rotation speed within a range of from about 10 rpm to about 50 rpm, more preferably within a range of from about 20 rpm to about 30 rpm, and most preferably of about 24 rpm.

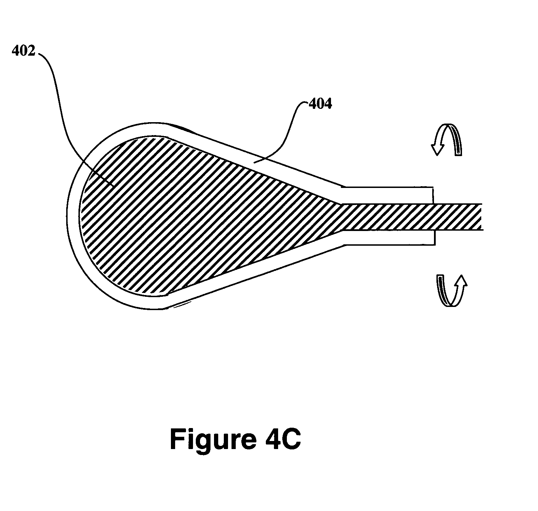

In order to further ensure even distribution of the liquid polymeric material along radial direction of the rotating mandrel 402, the rotating mandrel can be pivotally rotated to a third, substantially horizontal position, as shown in Figure 4C, before complete drying of the polymeric coating 402.

The complete drying of the polymeric coating 402 can be achieved by axially rotating the rotatory mandrel at said third, substantially horizontal position. Alternatively, the rotatory mandrel can be pivotally rotating to a fourth, fifth, ... position, before such complete drying, for further rotatory drying of the polymeric coating 402 under various surface flow conditions caused by the gravity.

Moreover, the multiple dipping and rotational drying steps as described hereinabove can be repeated in an alternating manner, so as to achieve an ultimate polymeric coating of sufficient thickness.

### (5) Curing:

After the polymeric coating of desired thickness and desired thickness gradient is completely dried, it is cured on the mandrel at an elevated temperature for a sufficiently long time. Preferably, such elevated curing temperature is with a range of from about 150°C to about 170°C, and such curing time is within a range of from about 45 to about 60 minutes.

After the curing, the solidified layer of polymeric material is removed from the rotatory mandrel to form an expandable polymeric medical article as described hereinabove.

### EXAMPLE

A SILBIONE® V40000 silicone composition comprising 35% solid silicone resin was dissolved in electronic grade xylene solvent to form a dipping solution having a viscosity within the range of about 700 to about 800 centipoises.

The dipping solution was poured into a dipping tank, and the viscosity of such dipping solution was further checked and adjusted to the desired range. The dipping solution was then allowed to equilibrate and filtered through the system for about half an hour.

The dipping temperature was strictly controlled to within a range of 75°F to 85°F. The dipping/drying process was conducted using an automated dip machine, namely a NAVIGATOR® Dipping System from ACC Automation Corporation U.S.A.

The dipping/drying protocol was set up as follows:

| **Step** | **Position Type** | **Dwell Time (seconds)** | **Axial Rotation Speed (rpm)** | **Mandrell Spinning** |
|---|---|---|---|---|
| Dipping | Vertical | 1 | 8 | Yes |
| Drying | Vertical | 180 | 8 | Yes |
| Dipping | Vertical | 1 | 8 | Yes |
| Drying | Vertical | 45 | 24 | Yes |
| Rotatory Drying | Inverted | 15 | 24 | Yes |
| Rotatory Drying | Horizontal | 450 | 24 | Yes |
| Rotatory Drying | Horizontal | 450 | 24 | Yes |
| ---- | Vertical | 0 | 0 | -- |
| Dipping | Vertical | 1 | 8 | Yes |
| Drying | Vertical | 180 | 8 | Yes |
| Dipping | Vertical | 1 | 24 | Yes |
| Drying | Vertical | 40 | 0 | No |
| Rotatory Drying | Inverted | 50 | 24 | Yes |
| Rotatory Drying | Horizontal | 900 | 24 | Yes |
| Rotatory Drying | Horizontal | 300 | 24 | Yes |
| Rotatory Drying | Horizontal | 900 | 24 | Yes |
| Rotatory Drying | Horizontal | 300 | 24 | Yes |

The whole dipping/drying program ran for about 1½ hours. After completion of the dipping/drying program, the mandrels were disengaged from the palette of the automated dipping machine, and loaded onto a T-bar.

The loaded T-bar was then placed in a preheated oven and was baked between 150-170°C for 30 to 90 minutes, preferably 45-60 minutes, so that the dimethyl siloxane material was completely cured. The hot mandrels were allowed to cool, and the neck area of each balloon formed was trimmed using a sharp razor blade, so as to form a neck of a minimum length of 0.5 inch.

A solution of 0.5% liquinox was prepared, and the balloons were filled with such 0.5% liquinox solution, which facilitated pulling of the balloons off the mandrels.

The balloons so formed had: (1) a neck thickness between 0.005--0.010 inch in average; (2) a transitional thickness between 0.005--0.007 inch in average; (3) an equatorial thickness between 0.003 to 0.005 inch in average; and (4) a dome thickness between 0.0025 to 0.0045 inch in average. The minimum neck length of the balloons so formed was 0.5 inch, and the pressure decay of such balloons was less than 3.05 mmHg/min.

Such balloons had no loose particulates or debris, no demolding stretch marks, no embedded fibers, no embedded air bubbles, and no jagged edge at the neck.

While the invention has been described herein with respect to various illustrative aspects, features and embodiments, it will be recognized that the invention is not thus limited, but that the present invention extends to and encompasses other features, modifications, and alternative embodiments, as will readily suggest themselves to those of ordinary skill in the art based on the disclosure and illustrative teachings herein. The claims that follow are therefore to be construed and interpreted as including all such features, modifications and alternative embodiments, within their spirit and scope.

## Claims

1. A dip-molded polymeric medical article, comprising an expandable enclosure including a distal portion, and a neck including a proximal portion, wherein said distal portion has first wall thickness before expansion, wherein said proximal portion includes an opening communicatively connected to said expandable enclosure and has second wall thickness, and wherein said second wall thickness is greater than said first wall thickness.

2. The dip-molded polymeric medical article of claim 1, selected from the group consisting of endometrial ablation balloons, catheter balloons, medication directing/delivery tubing, fluid storage/dispensing compartments, and protective covers.

3. The dip-molded polymeric medical article of claim 1, selected from the group consisting of endometrial ablation balloons, catheter balloons, condoms, surgical gloves, and tamponade device for control of postpartum hemorrhage.

4. The dip-molded polymeric medical article of claim 1, further comprising a transition portion and an equatorial portion between the proximal portion and the distal portion, wherein the transition portion is adjacent to said proximal portion, wherein the equatorial portion is adjacent to said distal portion, and wherein said polymeric medical article is **characterized by** a reverse thickness gradient that gradually increases from the proximal portion, along the transition portion and the equatorial portion, to the distal portion.

5. The dip-molded polymeric medical article of claim 1, where the first wall thickness is within a range of from about 6 to about 10 mils, and wherein the second wall thickness is within the range of from about 2.5 to about 5.5 mils.

6. The dip-molded polymeric medical article of claim 1, comprising at least one material selected from the group consisting of silicone, polyurethane, cis-1,4 polyisoprene, polyvinyl, SIS elastomer, SIBS elastomer, latex rubber, nitril rubber, and butal rubber.

7. The dip-molded polymeric medical article of claim 1, comprising a hypo-allergenic material.

8. The dip-molded polymeric medical article of claim 1, comprising a silicone material.

9. The dip-molded polymeric medical article of claim 1, comprising polydimethylsiloxane.

10. The dip-molded polymeric medical article of claim 1, **characterized by** a geometrically regular shape.

11. The dip-molded polymeric medical article of claim 10, having a shape selected from the group consisting of spherical, oval, cubic, rectangular, and polyhedral.

12. The dip-molded polymeric medical article of claim 1, **characterized by** a geometrically irregular shape.

13. The dip-molded polymeric medical article of claim 12, having a continuous and smooth surface morphology.

14. The dip-molded polymeric medical article of claim 1, **characterized by** a shape in substantially conformity with a body cavity or a body part.

15. A dip-molded polymeric medical article, comprising an expandable body member and at least one neck member connected thereto, wherein said at least one neck member is **characterized by**: (1) a cross-sectional diameter that is smaller than that of the expandable body member before expansion, and (2) a wall thickness that is greater than that of the expandable body member before expansion.

16. The dip-molded polymeric medical article of claim 15, selected from the group consisting of endometrial ablation balloons, catheter balloons, medication directing/delivery tubing, fluid storage/dispensing compartments, and protective covers.

17. The dip-molded polymeric medical article of claim 15, selected from the group consisting of endometrial ablation balloons, catheter balloons, condoms, surgical gloves, and tamponade device for control of postpartum hemorrhage.

18. The dip-molded polymeric medical article of claim 15, wherein the expandable body member comprises a transition portion adjacent to said neck member, an equatorial portion adjacent to said transition portion, and a dome portion that is most distal to the neck member, and wherein said polymeric medical article is **characterized by** a reverse thickness gradient that gradually increases from the neck member, along the transition portion and the equatorial portion, to the dome portion.

19. The dip-molded polymeric medical article of claim 15, where the wall thickness of the neck member is within a range of from about 6 to about 10 mils, and wherein the wall thickness of the dome portion of the expandable body member is within the range of from about 2.5 to about 4.5 mils.

20. The dip-molded polymeric medical article of claim 15, comprising at least one material selected from the group consisting of silicone, polyurethane, cis-1,4 polyisoprene, polyvinyl, SIS elastomer, SIBS elastomer, latex rubber, nitril rubber, and butal rubber.

21. The dip-molded polymeric medical article of claim 15, comprising a hypo-allergenic material.

22. The dip-molded polymeric medical article of claim 15, comprising a silicone material.

23. The dip-molded polymeric medical article of claim 15, comprising polydimethylsiloxane.

24. The dip-molded polymeric medical article of claim 15, **characterized by** a geometrically regular shape.

25. The dip-molded polymeric medical article of claim 24, having a shape selected from the group consisting of spherical, oval, cubic, rectangular, and polyhedral.

26. The dip-molded polymeric medical article of claim 15, **characterized by** a geometrically irregular shape.

27. The dip-molded polymeric medical article of claim 26, having a continuous and smooth surface morphology.

28. The dip-molded polymeric medical article of claim 15, **characterized by** a shape in substantially conformity with a body cavity or a body part.

29. An expandable polymeric medical article, formed by dipping a mandrel into a solution of a polymeric material for one or more times to apply a layer of said polymeric material onto said mandrel, and subsequently solidifying said layer of the polymeric material on said mandrel to form said expandable polymeric medical article, wherein said mandrel comprises a first end and a second end, said first end contacting said polymeric solution before said second end during the dipping process, wherein said expandable polymeric medical article comprises a first portion that is solidified on the first end of the mandrel, and a second portion that is solidified on the second end of the mandrel, and wherein said second portion is **characterized by** a wall thickness that is greater than that of the first portion before expansion of said expandable polymeric medical article.

30. A method for forming an expandable polymeric medical article, comprising the steps of:
(a) providing a liquid dip-molding composition comprising at least one polymeric material;
(b) providing a rotatory mandrel capable of axial rotation at an adjustable rotational speed, wherein said rotatory mandrel is capable of being pivotally rotated in at least one additional direction;
(c) dipping said rotatory mandrel into the liquid dip-molding composition so as to apply a layer of said polymeric material onto said rotatory mandrel;
(d) removing said rotatory mandrel from the liquid dip-molding composition;
(e) repeating steps (c) to (d) for one or more times, wherein between each dipping, said layer of polymeric material is partially dried on said rotatory mandrel at a substantially vertical position, with the rotatory mandrel axially rotating during such drying;
(f) pivotally rotating said rotatory mandrel in said additional direction to a substantially inverted position, and partially drying said layer of polymeric material thereat with said rotatory mandrel axially rotating during the drying;
(g) optionally, pivotally rotating said rotatory mandrel in said additional direction to a substantially horizontal position, and partially drying said layer of polymeric material thereat with said rotatory mandrel axially rotating during the drying;
(h) optionally, repeating steps (c) to (g) until said layer of polymeric material achieves a predetermined average thickness;
(i) curing said layer of polymeric material on said rotatory mandrel; and
(j) removing said layer of polymeric material from the rotatory mandrel to form said expandable polymeric medical article.

31. The method of claim 30, wherein the liquid dip-molding composition comprises at least one material selected from the group consisting of silicone, polyurethane, cis-1,4 polyisoprene, polyvinyl, SIS elastomer, SIBS elastomer, latex rubber, nitril rubber, and butal rubber.

32. The method of claim 30, wherein the liquid dip-molding composition comprises a liquid polymeric material.

33. The method of claim 30, wherein the liquid dip-molding composition comprises a solid polymeric material dissolved in a solvent.

34. The method of claim 30, wherein the liquid dip-molding composition comprises a solid polymeric material dispersed in a solvent.

35. The method of claim 30, wherein the liquid dip-molding composition is pseudoplastic.

36. The method of claim 30, wherein the liquid dip-molding composition comprises silicone.

37. The method of claim 30, wherein the liquid dip-molding composition comprises dimethyl siloxane dispersion.

38. The method of clam 37, wherein said dimethyl siloxane dispersion comprises xylene solvent.

39. The method of claim 37, wherein said dimethyl siloxane dispersion comprises silica filler selected from the group consisting of treated fumed silica and untreated fumed silica.

40. The method of claim 30, wherein said liquid dip-molding composition is **characterized by** a viscosity within a range of from about 400 centipoises to about 1000 centipoises.

41. The method of claim 30, wherein said liquid dip-molding composition is **characterized by** a viscosity within a range of from about 500 centipoises to about 900 centipoises.

42. The method of claim 30, wherein a 3-axises dipping system comprising multiple rotatory mandrels is used for dipping and drying.

43. The method of claim 42, wherein said dipping system comprises 30 rotatory mandrels.

44. The method of claim 43, wherein said dipping system comprises more than 100 rotatory mandrels.

45. The method of claim 30, wherein the dipping is conducted with the rotatory mandrel axially rotating at an axial rotation speed within a range of from about 1 rpm to about 20 rpm.

46. The method of claim 30, wherein the dipping is conducted with the rotatory mandrel axially rotating at an axial rotation speed within a range of from about 5 rpm to about 10 rpm.

47. The method of claim 30, wherein the partial drying of said layer of polymeric material between each dipping is conducted at a sufficient temperature for a sufficient duration, so as to obtain a surface layer of sufficient viscosity for adhesion of a next layer of polymeric material thereon during subsequent dipping.

48. The method of claim 30, wherein the partial drying between each dipping is conducted at a temperature within a range of from about 70°F to about 90°F.

49. The method of claim 30, wherein the partial drying between each dipping is conducted at a temperature within a range of from about 75°F to about 85°F.

50. The method of claim 30, wherein the partial drying between each dipping is conducted for a duration within a range of from about 150 seconds to about 200 seconds.

51. The method of claim 30, wherein the partial drying between each dipping is conducted for a duration within a range of about 180±5 seconds and at a temperature within a range of from about 75°F to about 85°F.

52. The method of claim 30, wherein the partial drying of said layer of polymeric material at said substantially inverted position is conducted for a sufficiently long duration, so as to distribute said polymeric material over the rotatory mandrel for the purpose of forming an expandable polymeric medical device with a neck member and a body member, wherein said neck member has a wall thickness that is greater than that of said body member.

53. The method of claim 30, wherein the partial drying of said layer of polymeric material at said substantially inverted position is conducted for a duration within a range of from about 10 to about 20 seconds.

54. The method of claim 30, wherein the partial drying of said layer of polymeric material at said substantially inverted position is conducted with the rotatory mandrel axially rotating at an axial rotation speed that is higher than that used for the partial drying between dipping.

55. The method of claim 30, wherein the partial drying of said layer of polymeric material at said substantially inverted position is conducted with the rotatory mandrel axially rotating at an axial rotation speed within a range of from about 10 rpm to about 50 rpm.

56. The method of claim 30, wherein said layer of polymeric material is cured at an elevated curing temperature within a range of from about 150°C to about 170°C, and for a duration within a range of from about 30 minutes to about 90 minutes.
